Europäisches Patentamt

European Patent Office

Office européen des brevets

(11) Publication number: **0 450 861 B1**

# EUROPEAN PATENT SPECIFICATION

(45) Date of publication of patent specification: **19.07.95**  (51) Int. Cl.[6]: **C07C  1/04**, C07C 1/06,
B01J 23/74

(21) Application number: **91302711.6**

(22) Date of filing: **27.03.91**

(54) **Process for hydrocarbon synthesis using slurry Fischer-Tropsch process with Co/TiO2 catalyst.**

(30) Priority: **04.04.90 US 504747**

(43) Date of publication of application:
**09.10.91 Bulletin  91/41**

(45) Publication of the grant of the patent:
**19.07.95 Bulletin  95/29**

(84) Designated Contracting States:
**BE DE FR GB IT NL**

(56) References cited:
**EP-A- 0 188 304**
**EP-A- 0 220 343**
**EP-A- 0 313 375**
**US-A- 4 279 830**

(73) Proprietor: **EXXON RESEARCH AND ENGI-
NEERING COMPANY
P.O.Box 390,
180 Park Avenue
Florham Park,
New Jersey 07932-0390 (US)**

(72) Inventor: **Arcuri, Kym Brian
15346 Schnebelen Drive
Baton Rouge, LA 70817 (US)**

(74) Representative: **Somers, Harold Arnold et al
ESSO Engineering (Europe) Ltd.
Patents & Licences
Mailpoint 72
Esso House
Ermyn Way
Leatherhead,
Surrey KT22 8XE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

This invention relates to a hydrocarbon synthesis process.

BACKGROUND OF THE INVENTION

The hydrocarbon synthesis process involves the catalytic hydrogenation of carbon monoxide with hydrogen to form higher hydrocarbons, preferably $C_5$ + hydrocarbons. Hydrogen and carbon monoxide, synthesis gas, are contacted with a suitable catalyst at appropriate reaction conditions, usually elevated temperatures and pressures, to produce the desired hydrocarbons. Catalytic materials usually include Group VIII metals, particularly iron, cobalt, ruthenium, and nickel supported on a porous inorganic oxide support, such as the oxides of Group IIIA, IV, VA. Promoter materials can be employed, e.g., rhenium, hafnium and other lanthanide metals, zirconium and other Group VIII metals, as well as the metals of Groups IA, IB and IIA.

The results obtained with a particular catalyst may vary considerably from results obtained from any other catalyst system. Also, the type of reaction system in which the catalyst is placed can affect the results. The two extremes or poles of reaction system are the plug flow system, exemplified by a fixed catalyst bed where back mixing is either non-existent or considerably minimized, and the well stirred system where complete back mixing is effected, exemplified by fluidized beds and in liquid phase systems by the well stirred or fully back mixed reactor system.

In any hydrocarbon synthesis reaction the rate of CO conversion and product selectivity depends on the partial pressure of the reactants, hydrogen and carbon monoxide, and in some cases the products, e.g., water, olefins, in contact with the catalyst. Thus, the mixing characteristics of the reactor become critical in determining catalyst performance since these characteristics will determine the gas phase composition (and therefore, the partial pressure of the reactants) at any particular point in the reactor.

In the fully back mixed reactor or CSTR, the composition of reactants (gaseous) and products (liquids and gases) and condition of the catalyst at any one point in the reactor is the same as that at any other point in the reactor. Achieving this ideal state of mixing can be accomplished with mechanical stirring devices. The reactant concentration or gas partial pressure of hydrogen and carbon monoxide govern catalyst performance by providing the driving force of the reaction and sets the carbon monoxide conversion occurring in the reactor. Thus, even though pure synthesis gas feed is entering the reactor, catalyst performance is driven by the reactant gas phase concentration corresponding to the reactant gas phase concentration exiting the reactor. This system, fully back mixed, provides maximum selectivity to desired products at the expense of productivity.

The other extreme of reactor mixing occurs in the plug flow reactor where the catalyst is stationary or relatively stationary relative to the flow of reactants (gaseous) and products (liquids and gases). The synthesis gas feed undergoes reaction as it enters the reactor and the reaction continues as the unreacted synthesis gas proceeds through the reactor. Thus, the concentration of, and partial pressure of hydrogen and carbon monoxide, decreases along the path of the reactor and, therefore, the driving force of the reaction also decreases as the concentration of liquid and gaseous hydrocarbon products as well as $H_2O$ by-product increases. Thus, the catalyst at the exit portion of the plug flow reactor never sees fresh feed. The plug flow system provides maximum productivity at the expense of selectivity.

The important difference between the fully backed mixed (CSTR) and plug flow systems is the difference in the gas phase reactant concentration that provides the kinetic driving force for the reaction. In the fully back mixed system the reactant concentration is the same at any point in the reactor; in the plug flow system the reactant concentration steadily decreases along the path of the catalyst bed from inlet to outlet and the reaction rate is obtained by integrating the rate function from inlet to outlet.

Now, because the reactant concentration at any point in a CSTR system always corresponds to outlet conditions the productivity in a fully back mixed system will always be lower than the productivity in a plug flow system particularly in hydrocarbon synthesis where catalysts frequently have a positive rate dependence on hydrogen partial pressure and lesser or negative rate dependent on CO partial pressure. This is axiomatic because the outlet reactant concentration of a plug flow reactor is always the lowest reactant concentration in the reactor. Reactant concentrations at any point upstream of the outlet will be higher than at the outlet and the kinetic driving force will, therefore, be higher upstream of the outlet.

Reactor systems exhibiting plug flow and well stirred characteristics are the extremes of reactor performance. In practice, plug flow reactors may exhibit some back mixed traits and back mixed reactors may exhibit some plug flow traits. Deviations from the ideal systems are due to the dispersion of the reactant gases in the reactor. Insuring complete back mixing is a function of the mechanical energy imparted to the system. Reactor geometry can affect back mixing and low length to diameter ratios, less than about 3, promote back mixing in plug flow reactors. However, with higher energy inputs reactors with greater length to diameter ratios can achieve complete back mixing, too. Conversely, plug flow is favored by high length to diameter ratios. The degree of non-ideal back mixing that can occur in a plug flow reactor can be represented by the Peclet Number, $N_{pe}$ which is equal to $LU/\Delta$, where L is the reactor length (or catalyst size), U is the gas velocity, and $\Delta$ is the dispersion coefficient.*

High $N_{pe}$ indicates plug flow while low $N_{pe}$ indicates CSTR. By definition, the dispersion coefficient for an ideal CSTR is infinity and $N_{pe}$ approaches zero.

While plug flow and CSTR represent the ideal extremes of reactor systems, classical chemical engineering principles define a continuous function between plug flow and CSTR. That is, as a system is less and less ideal plug flow it will move in a continuous fashion towards CSTR; similarly, a system exhibiting less and less back mixing will move in a continuous fashion towards plug flow. Therefore, in accordance with classical chemical engineering principles, a given reactor system must follow the continuous function between ideal plug flow and ideal CSTR and cannot fall outside this function. Thus, classical chemical engineering principles teach that productivity reactor with decreasing plug flow characteristics can be no higher than the productivity in the starting point plug flow reactor. Conversely, the selectivity in a reactor with decreasing back mixed characteristics can be no greater than the starting point back mixed reactor.

A number of review articles and patents have been published in which both slurry and fixed bed hydrocarbon synthesis reactions with various catalysts have been prepared. Among the articles are: Carrol, E.E. et al, Quarterly Tech. Prog. Report for Period 4/1-6/30/86, DDE Report #DE87 006115, Contract No. DE-AC22-84 PC 70030; Hall, C.C. et al J. Inst. Pet. 38 845 (1952); Results from the Rheinpreussen-Koppers Demonstration Plant, presented by Kolbel, N. and Ralek, M., Catalyst Rev. Sci. Eng. 21 (2) 225 (1980); European Patent Application 0-194-552; U.S. Patent #4,619,910; Fujimoto, Faud Kajaka, M, Bull. Chem. Soc. Jpn. 60 2237 (1987); Satterfield, C.N. et al Ind. Eng. Chem. Fund. 1985, 24, 450-454; Dry, M.E. Catalysis Sci & Tech., Vol 1, ed. J.R. Anderson & M. Boudart, Springer Verlag (1981); Van Vuuren, Council for Scientific & Ind. Res. Rep; LENG 432 (1982).

US-A-4 279 830 discloses carrying out a Fisher-Tropsch synthesis gas conversion in a liquid phase bubble column reactor with suspended catalyst particles. No details are given of the nature of the reactor. The catalyst particles employed are iron with or without an alkali metal promoter.

EP-A-220 343, an earlier document of the present Applicants, discloses a catalyst suitable for synthesis gas conversion comprising cobalt, or cobalt/rhenium, on a titania support. Syngas conversion employs the catalyst in powdered form in fixed beds.

According to the present invention there is provided a hydrocarbon synthesis process comprising reacting hydrogen and carbon monoxide, in the presence of catalyst containing cobalt on a titania support or a titania-containing support, in a slurry bubble column with the Peclet number being at least 3 but not greater than 10.

This invention provides a process wherein catalyst stability is virtually constant (i.e., there is little if any catalyst deactivation with time), $CH_4$ selectivity is quite low, while at the same time CO productivity is quite high, and $CO_2$ selectivity is low.

For purpose of this invention, the following definitions apply:

CO productivity is the moles CO converted per gram of catalyst - hour;

$CO_2$ selectivity is the moles $CO_2$ produced per 100 moles CO converted;

$CH_4$ selectivity is the moles $CH_4$ produced per 100 moles CO converted;

$C_5$ + selectivity is the moles $C_5$ + produced per 100 moles CO converted; and

Catalyst stability is the percent loss in CO productivity per day.

The invention provides a hydrocarbon synthesis system that allows the productivity of fixed bed reactors and the selectivity of back mixed reactors. The invention requires the combination of a cobalt/titania catalyst system and a defined bubble column reactor. A bubble column reactor is a slurry system: it operates in the liquid phase, but it is not a well stirred reactor. Therefore, the results, according to engineering principles should show lower productivity than a fixed bed and selectivity approaching that of a well stirred reactor. In fact, the defined bubble column in combination with with a cobalt/titania reactor system shows essentially at least the productivity of fixed bed reactors and the selectivity of well stirred reactors.

* Dispersion coefficient is described by Kobel and Ralek in "The Fischer-Tropsch Synthesis in the Liquid Phase", Catal.Rev.Sci.Eng., 21(2), 225-274 (1980).

The catalyst is slurried in a liquid medium, preferably the indigenous hydrocarbon wax produced by the hydrocarbon synthesis process, and the reaction takes place in a bubble column that is not a well stirred reactor, that is, $N_{pe}$ is greater than 3 but less than 10. In a preferred embodiment, the catalyst is promoted with rhenium. In another preferred embodiment the slurry liquid comprises hydrocarbon synthesis waxes boiling, at atmospheric pressure, above about 700°F (371°C), more preferably between 700°F and 1025°F (371 to 551°C). Following this invention allows obtaining a relative productivity in a slurry bubble column at least as great as that obtained in a plug flow system, and ideally, a selectivity to $C_5 +$ hydrocarbons at least as great as that obtained in a fully back mixed system.

DESCRIPTION OF THE DRAWINGS

Figure 1 shows the relationship between relative CO productivity and increasing degree of back mixing, that is from 0% back mixing in a plug flow reactor to 100% back mixing in a fully back mixed reactor. Bubble column results with a cobalt-rhenium/titania catalyst are far in excess of predicted results.

Figure 2 shows the same relationship for a cobalt-rhenium/titania-alumina binder catalyst.

Reaction conditions for hydrocarbon synthesis processes are generally well known. However, for this invention temperatures may range from 160°C to 360°C, preferably 190°C to 230°C, and more preferably 190°C to 220°C. Pressures are normally above about 80 psig (0.55 MPa), preferably 80-600 psig (0.55 to 4.14 MPa), more preferably 150 psig to 350 psig (1.03 to 2.41 MPa). Increasing temperature generally increases CO productivity, all other things being equal, however, methane selectivity also tends to increase and catalyst stability diminishes. Thus, while CO conversion increases, the yield of desirable liquid products, e.g., $C_5 +$, $C_{10} +$, may not be as great.

Hydrogen to carbon monoxide ratios may vary widely, also. Although the stoichiometric $H_2$:CO ratio for Fischer-Tropsch reactions approaches 2.1:1, most slurry phase processes use relatively low $H_2$:CO ratios. For example, U.S. 4,681,867 discloses preferred hydrogen: carbon monoxide operating ratios of 1:2 to 1:1.4. Slurry-type processes generally employ $H_2$:CO ratios of 1.0 or less and is evidence of either a less active catalyst or mass transfer limitations on CO entering the liquid phase. This invention is not limited to low ratios of $H_2$:CO and, in fact, $H_2$:CO ratios at or near the stoichiometric ratio are preferred. Thus, $H_2$:CO ratios may range from 1.5:1.0 to 2.5:1, more preferably 1.2:1 to 2.2:1.

The operation of bubble columns has been described generally in several prior art references, e.g., the Van Vuuren and Kolbel and Ralek references mentioned above, South African patent application 85/5317, U.S. patent 4,681,867, and EP-A-313 375. However, as previously mentioned, and in accordance with this invention, the bubble column is operated within a particular Peclet number range, i.e., greater than about 3 and less than about 10.

Peclet numbers were calculated, where possible and making reasonable assumptions where appropriate, from several prior art references regarding bubble column operation as shown below:

| | |
|---|---|
| Deckwer et al, Chem. Eng. Sci., 29 2177 (1974) | $N_{pe}$ = 0.39 |
| Kato et al, J. Chem. Eng. Japan, 5 112 (1972) | $N_{pe}$ = 0.35 |
| Joshi, Chem. Eng. J., 24 213 (1982) | $N_{pe}$ = 20 |
| Field et al, Trans. Inst. Chem. Engrs., 58 228 (1980) | $N_{pe}$ = 32 |
| Mangartz et al, Verfahreustechnik, 14 40 (1980) | $N_{pe}$ = 20.7 |
| EP-A-313 375 | $N_{pe}$ = 94 |

In a bubble column reactor the catalyst is suspended and mixed by the motion induced by the rising gas bubbles. Feed gas is introduced into the bottom of the reactor and rises through the suspended catalyst as individual bubbles, thereby creating mixing by displacing both liquid and solids.

Hydrocarbon synthesis is a positive order reaction for hydrogen and at best zero order for carbon monoxide. Kinetics of the reaction and product selectivity then depend on the partial pressure of the reactants, hydrogen and carbon monoxide. The table below depicts a qualitative assessment of experimental facts, predictions based on engineering kinetics, and actual observance. P' denotes productivity, S denotes selectivity to $C_5 +$ hydrocarbons, $\Delta$ is dispersion, and 1 is back mixed, 2 is fixed bed, and 3 is bubble column, PP is partial pressure, and P is total pressure.

| | Experimental Assumptions Back Mixed | Fixed Bed | Predictions Bubble Column | Actual Bubble Column |
|---|---|---|---|---|
| $P'$ | low | high | $P'_2 > P'_3 > P'_1$ | $P'_3 \geq P'_1$ |
| $S$ | high | low | $S_2 < S_3 < S_1$ | $S_3 \geq S_2$ |
| $\Delta$ | $\infty$ (1) | 0 | intermediate | intermediate towards fixed bed |
| $\dfrac{PP_{CO}}{PP_{H_2}}$ | low | high | $PP_{CO_1} < PP_{CO_3} < PP_{CO_2}$ | $PP_{CO_3} - PP_2$ |
| $P_{CO+H_2}$ (i.e., total pressure of $CO + H_2$) | high | low | intermediate | intermediate |

(1) dispersion at full back mixing approaches infinity

Operation of bubble columns requires controlling variables other than temperature, pressure, and synthesis gas ratio. Thus, the liquid medium used for slurrying the catalyst can be generally any material that will be liquid at operating temperatures and pressures, maintain the catalyst in suspension, relatively or largely inert at reaction conditions, and a good solvent for carbon monoxide and hydrogen. Suitable materials can be saturated paraffins or olefinic polymers boiling above about 300°F (149°C), preferably at least about 550°F (288°C). Additionally, suitable slurry media can be Fischer-Tropsch waxes produced by any Fischer-Tropsch catalyst but particularly hydrocarbon materials produced using a cobalt on titania supported catalyst, and most preferably those liquids that boil above between about 700°F (371°C), still more preferably at 700°F-1025°F (371 to 552°C). As the reaction proceeds, the indigenous wax, that is, the wax produced by the process of this invention will replace the material used for startup purposes, and eventually the slurry medium is most preferably substantially completely, e.g., at least 90%, indigenous wax.

5

Oxygenates tend to promote foaming and the slurry medium should contain no more than about 2 wt% oxygenates. Catalysts such as cobalt or cobalt-rhenium on titania or a titania containing support produce very low levels of oxygenates and are ideally suited for operation in this process.

The solids loading, that is, volume of catalyst per volume of slurry or diluent is up to about 50% and preferably ranges from 10% to 40%. The solids may range from powders to discreet particles, for example, from 5 microns to 1 mm, preferably 10 microns to 200 microns, more preferably from 20 to 100 microns. (Sizes are expressed as mean particle size, e.g., because particles usually have a size distribution.)

Feed gas, which may be diluted with some inert gas, i.e., less than about 30 vol%, preferably less than about 20 vol%, such as nitrogen or $CO_2$, is usually introduced into the bottom of the reactor and bubbles, through to the top of the reactor. Use of higher levels of diluent gas will not only limit the maximum amount of product formed per total volume of gas fed to the reactor, but also require costly separation steps to remove the diluent from valuable $H_2$ and CO reactants. Feed gas velocity is usually as high as possible while avoiding foaming which results when gas bubbles do not disengage from the liquid. Thus, stable operation occurs when the gas contained in the slurry does not increase with time or increases only slightly. Foaming occurs when gas holdup time increases with time. Gas holdup can be defined as the fraction of gas in the three phase slurry mixture.

Suitable gas velocities are those that result in suspending the particles in the liquid medium and are usually greater than about 2 cm/sec.

Generally, a disengagement zone is provided where catalyst and product are separated. Separation may be effected in a quiescent zone where catalyst settles out of the slurry and product. Filtration can also be used and may be external or internal. With external filtration, the catalyst and slurry medium are recovered and recycled to the reaction zone. With internal filtration, liquid is removed as quickly as it is formed maintaining the liquid level at a somewhat constant volume in the reactor. Magnetic separation of catalyst from liquid may also be used.

As mentioned earlier, length to diameter ratios have an effect on determining the Peclet number, and greater length/diameter ratios tend toward plug flow as smaller length/diameter ratios tend toward increased back mixing. One skilled in the art can design suitable bubble columns by using the Peclet Number equation:

$$\frac{\Delta}{uL} \frac{d^2 X_{CO}}{dZ^2} - \frac{d\ C_A}{dZ} - \frac{(-r)}{v/Q} = 0$$

wherein $\Delta/uL$ is the reciprocal of the Peclet Number and L is the length of the reactor.

u       is the feed gas velocity
$\Delta$       is the dispersion coefficient
$X_{co}$       is the mol fraction CO or CO partial pressure
$C_A$       is
Z       is
-r       is the molar rate of CO consumption
Q       is gas flow rate, i.e., $H_2$ + CO + diluent per unit time
V       is reactor volume

and the dispersion coefficient $\Delta$ is obtained from experimentation or texts and will be easily available to one skilled in the art.

We have found that only the catalyst of this invention provides results which have the attributes of plug flow productivity and fully back mixed selectivity.

The catalyst comprises cobalt on a primarily titania support. Cobalt is present in amounts sufficient to be catalytically active for Fischer-Tropsch synthesis. Usually cobalt concentrations may be at least about 5 wt%, preferably 5 to 45 wt%, more preferably about 10-30 wt%. The cobalt or cobalt and promoter is dispersed on titania or a primarily titania support. Preferably the titania is in the rutile phase, that is the rutile/anatase ratio is at least about 2:3, preferably 3:2, more preferably at least 4:1 to completely rutile. The ratio is determined in accordance with ASTM D 3720-78: Standard Test Method for Ratio of Anatase to Rutile in Titanium Dioxide Pigments By Use of X-Ray Diffraction. The support is at least about 50% titania, preferably about 80% titania and may contain other inorganic oxides suitable as catalyst supports. Materials such as alumina, silica, and zirconia may be employed in amounts ranging from 0.5 to 10 wt% as a binder material. Alumina and silica are preferred, alumina is most preferred.

The catalyst may also contain another metal that is either active as a Fischer-Tropsch catalyst, e.g., Group VIII non-noble metals, such as ruthenium, or a promoter metal, such as, rhenium, hafnium, zirconium, cerium, thorium or thoria, and uranium. Promoter metals are usually present in amount of at least 0.05:1 relative to cobalt, preferably at least 0.1:1, and most preferably 0.1:1 to 1:1. Rhenium is a preferred promoter.

The catalytically active metal, or metals, preferably cobalt or cobalt promoted or modified with an additional metal, or metals, can be dispersed upon a calcined titania or titania-binder support in a manner which will distribute the metal, or metals, essentially uniformly throughout the support particles from the center outwardly, or essentially upon the peripheral surface of the particle. Catalysts can be prepared by the several techniques known in the art for the preparation of catalysts, generally. In distributing the metal, or metals, uniformly throughout the support, the metal, or metals, can be deposited on the support from solution in preselected amounts to provide the desired absolute amounts, and weight ratio of the respective metal, or metals. Suitably, e.g., cobalt, or cobalt and ruthenium or cobalt and rhenium, are composited with support by contacting the support with a solution of a cobalt-containing compound, or salt, or a rhenium-containing compound, or salt, followed by impregnation of the other component. Optionally, the cobalt, or cobalt and rhenium can be co-impregnated upon the support. The cobalt used in the impregnation can be any organometallic or inorganic compound which decomposes to give cobalt oxides upon calcination, or can be reduced directly to cobalt with hydrogen, such as cobalt nitrate, acetate, acetylacetonate, naphthente or carbonyl. Likewise the rhenium compound used in the impregnation can be any organometallic or inorganic compound which similarly decomposes, e.g., perrhenic acid, ammonium perrhenate. The amount of impregnation solution used should be sufficient to completely immerse the carrier, usually within the range from 1 to 20 times of the carrier by volume, depending on the metal, or metals, concentration in the impregnation solution. The impregnation treatment can be carried out under a wide range of conditions including ambient or elevated temperatures. On the other hand, the catalytically active cobalt component is most preferably dispersed and supported upon the peripheral or outer surface of calcined titania-binder particles as a thin catalytically active surface layer ranging in average thickness from 20 microns to 200 microns when employing particles of about 1 mm or above. However, slurry catalysts are usually powders as discribed above (even though larger particles can be slurried) and in that case the active surface layer may be 2 to 20 microns, preferably 2-10 microns. The feature of a high cobalt metal loading in a thin catalytically active layer located at the surface of the particles can optimize the activity, selectivity and productivity of the catalyst in producing liquid hydrocarbons from synthesis gas, while minimizing methane formation in fixed bed or plug flow reactors.

The surface impregnated catalysts can be prepared by spray techniques where a dilute solution of a cobalt compound, alone or in admixture with a promoter metal compound, or compounds, as a spray is repetitively contacted with hot support particles. The support particles are maintained at temperatures equal to or above about 140°C when contacted with the spray, and suitably the temperature of the support particles ranges from about 140°C up to the decomposition temperature of the cobalt compound, or compounds in admixture therewith; preferably from 140°C to 190°C. The cobalt compound employed in the solution can be any organometallic or inorganic compound which decomposes to give cobalt oxide upon initial contact or upon calcination, such as cobalt nitrate, cobalt acetate, cobalt acetylacetonate, cobalt naphthenate or cobalt carbonyl. Cobalt nitrate is especially preferred while cobalt halide and sulfate salts should generally be avoided. The cobalt salts may be dissolved in a suitable solvent, e.g., water, organic or hydrocarbon solvent such as acetone, methanol, pentane. The total amount of solution used should be sufficient to supply the proper catalyst loading, with the film being built up by repetitive contacts between the support and the solvent. The preferred catalyst is one which consists essentially of cobalt, or cobalt and promoter, dispersed upon the support, especially a support the titania portion of which is comprised of rutile. Suitably, the hot support particles are contacted with a spray which contains from 0.05 g/ml to 0.25 g/ml, preferably from 0.10 g/ml to 0.20 g/ml, of the cobalt compound or cobalt compound plus the compound containing the promoter metal, generally from at least 3 to 12 contacts, preferably from 5 to 8 contacts, with intervening drying and calcination steps being required to form surface films of the required thicknesses. The drying step is generally conducted at temperatures ranging above about 20°C, preferably from 20°C to 125°C, and the calcination steps at temperatures ranging above 150°C to 500°C.

For practical operations, bubble column operation should have CO conversions of at least about 40%, preferably at least 50%, and $C_5$ + selectivities of at least 70%, preferably at least 85%.

Accepted chemical engineering principles teach that a bubble column reactor must lie between the mixing regimes defined by plug flow and full back mixing. However, the cobalt or cobalt promoted, particularly with rhenium/titania, catalyst exhibits the productivity of a plug flow system without the selectivity debits that these principles predict.

## EXAMPLES 1-8

Several experiments were conducted in plug flow (fixed bed), bubble column, and fully back mixed reactors (CSTR).

The results are shown in Table I below.

**TABLE I**

| Reactor | Exp # | T, avg °C | P, psig (MPa) | H₂/CO Feed Ratio | (1) Feed Gas Rate | % CO Conv | CO₂ | Product Selectivities | | | Productivity | Wt% Co | Wt% Re |
|---|---|---|---|---|---|---|---|---|---|---|---|---|---|
| | | | | | | | | C1 | C2-C4 | C5+ | | | |
| Fixed | 1 | 214 | 280 | 2.07 | 0.909 | 89.6 | 0.2 | 7.03 | ~4 | 89 | 0.0110 (2) | 12 | 0.5 |
| Fixed | 2 | 218 | 280 | 2.07 | 0.909 | 92.2 | 0.5 | 7.57 | ~4.3 | 88.1 | 0.0110 (2) | 12 | 0.5 |
| Fixed | 3 | 222 | 280 | 2.08 | 0.909 | 91.0 | 0.67 | 10.9 | ~5.4 | 83.7 | 0.0110 (2) | 12 | 0.5 |
| Fixed | 4 | 216 | 280 | 2.1 | 3.00 | 49 | <.1 | 8.1 | 6.4 | 85.5 | 0.0430 | 12 | 1 |
| CSTR | 5 | 210 | 280 | 2.1 | 1.360 | 47.9 | <0.05 | 4 | 4.1 | 91.9 | 0.0090 | 12 | 0.5 |
| CSTR | 6 | 215 | 280 | 2.1 | 0.909 | 65.8 | <0.05 | 3 | 4 | 93 | 0.0081 | 12 | 0.5 |
| Bubble | 7 | 214 | 280 | 2.02 | 2.0 | 81.2 | 0.19 | 3.2 | 2.7 | 93.9 | 0.0420 | 12 | 1 |
| Bubble | 8 | 190 | 280 | 2.08 | 1.40 | 41.5 | <0.05 | 2.3 | 3.3 | 94.4 | 0.0510 | 12 | 1 |
| Bubble | 9 | 230 | 280 (1.93) | 2.07 | 2.2 | 91 | 1.0 | 6.9 | 3.9 | 88.2 | 0.051 | 12 | 1 |

Notes:

(1) liters syn gas @ 60°F/1 atm/gm cat-hour (15.5°C/0.10 MPa/gm cat-hour)

(2) used catalyst

8

The Peclet Number of the fixed bed runs were all well above 10 and usually above about 30. The back mixed reactor runs had Peclet Numbers approaching 0. The bubble column runs all had Peclet Numbers ranging between 3 and 10.

In hydrocarbon synthesis, the goal is avoiding methane formation, methane being virtually useless, other than a fuel, and having the lowest product value. In the plug flow (fixed bed) reactors, methane selectivity was relatively high, 7-10 wt%, while in the fully back mixed reactors (CSTR) which favor product selectivity, methane was relatively low, 3-4 wt%. However, the bubble column reactor exhibited methane selectivities in the same order as the CSTR, when accepted principles would have predicted methane selectivites somewhere between CSTR and plug flow.

Run 4 shows the best productivity for a fixed bed reactor but methane selectivity remains relatively high. Runs 5 and 6 represent a fully back mixed reactor system and productivities are relatively low. In fact, the productivity for Runs 1-3 even with a used catalyst, is higher than that for a back mixed reactor with fresh catalyst, thereby highlighting the effect of classical engineering principles.

Now, bubble column Runs 7, 8, and 9 all exhibit a productivity at least as high as that for the fixed bed Runs 1-4. However, contrary to what would have been predicted $C_5$+ selectivities are higher than for plug flow/fixed bed systems and methane selectivities are in the range of the back mixed systems.

Run 9 shows high productivity with equivalent $C_5$+ relative to runs 1-4. However, the methane selectivity is relatively high as well. This can be readily accounted for by the temperature of Run 9 which was 230°C. While higher temperatures generally favor increased productivity, the selectivity is generally more to gases, e.g., methane, than to higher hydrocarbons.

Thus, the productivity of the bubble column reactors was much higher than the productivity of the CSTR runs. Thus, the bubble column exhibited fully back mixed selectivity with increased productivity.

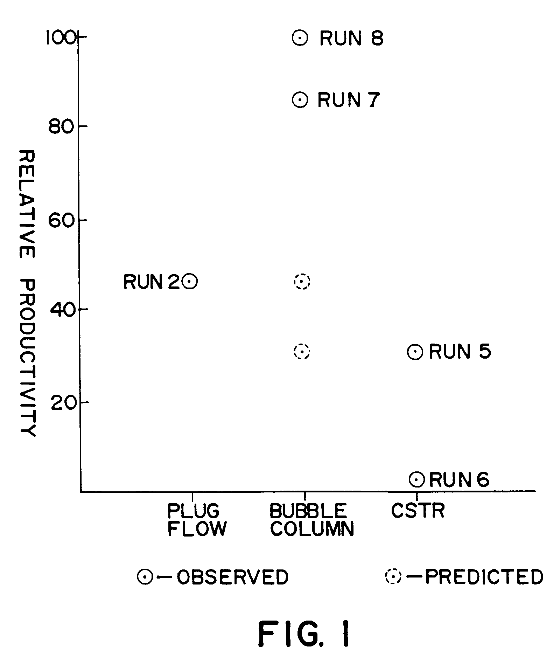

The plug flow (fixed bed) reactor productivity of Run 2 is shown on Figure 1 above the fixed bed point.*The fully back mixed points are Runs 5 and 6. Because slurry phase hydrocarbon synthesis reactions are negative order with respect to CO, or at best, zero order, the best possible result in a bubble column would be equivalent productivity or a horizontal line from point 2 (zero order reaction). For negative order reactions, chemical engineering principles predict a line of negative slope from fixed bed (point 2) to fully back mixed (point 4 or point 5) with bubble column productivity falling on the lines of negative slope. The dotted circle represents the predicted point for bubble column operation for averaging Runs 5 and 6. However, actual bubble column results are shown by points 7 and 8, both of which have methane selectivities of the same order as back mixed Runs 5 and 6.

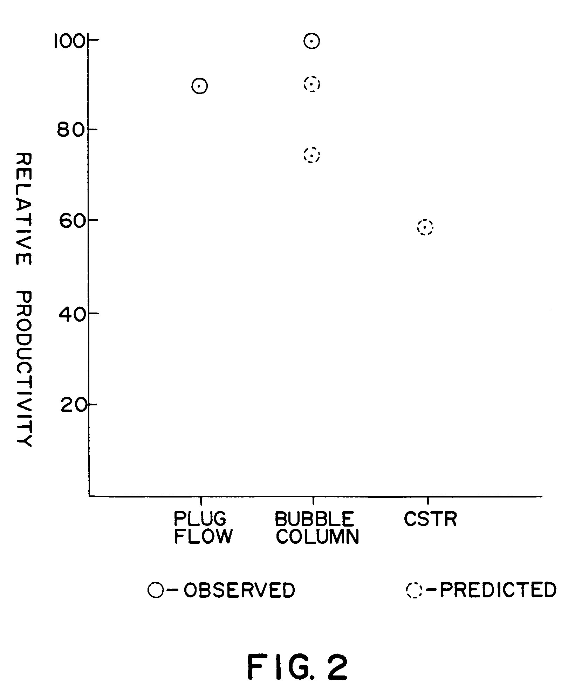

Figure 2 shows actual results for a plug flow (fixed bed) reactor at 215°C using 12 wt% CO, 1 wt% rhenium on a titania-3 wt% alumina binder support. The negative or zero order reaction rate for a fully back mixed system suggests a predicted value shown by the dotted circle above "CSTR", and therefore, bubble column results should be no greater than that shown by the dotted circles above "Bubble Column". However, the actual result showed a higher productivity than for plug flow with a methane selectivity of only 2.9 wt% versus a methane selectivity of 9.2 wt% for the plug flow reactor.

EXAMPLE 9

Experiments were conducted using different liquids as the slurry medium. Results are shown in Table II below.

The results were obtained using a stirred autoclave reactor in a semi-batch mode wherein liquid product was periodically removed through an internal filter element. When the start-up liquid was light (700°F-) (371°C-), volumetric activity was good but methane selectivity was relatively high. At the other end of the range, with 1025°F+ (552°C+) material, the methane selectivity was low but activity was relatively poor. The intermediate liquid, 700-1025°F (371 to 552°C), gave both relatively low methane selectivity and relatively high volumetric activity.

The six hour period was selected to allow the gas phase composition to reach steady state; after 48 hours the initial liquid was diluted with about 25 wt% of liquids produced by hydrocarbon synthesis.

* The graphs of the drawings show relative productivity values (productivity normalized to weight of catalyst), since productivity-comparisons with different processes cannot easily be made.

TABLE II

| INITIAL SOLVENT CUT (°F) (°C) | 500°F (C-16) (260°C) | 300-700°F (149 to 371°C) | 700-1025°F (571 to 552°C) | 700°F+ (371°C+) | 1025°F+ (552°C+) |
|---|---|---|---|---|---|
| **6 Hours On Syngas** | | | | | |
| % CO CONVERSION | 40 | 62 | 62 | 62 | 43 |
| CH₄ SELECTIVITY | 3.5 | 2.2 | 1.5 | 1.5 | 3.4 |
| RELATIVE VOLUMETRIC ACTIVITY | 64.5 | 100 | 100 | 100 | 69.3 |
| **48 Hours On Syngas** | | | | | |
| % CO CONVERSION | 48 | 50 | 58 | 58 | 48 |
| CH₄ SELECTIVITY | 2.8 | 2.2 | 1.3 | 1.6 | 1.6 |
| RELATIVE VOLUMETRIC ACTIVITY | 82.7 | 86.2 | 100 | 100 | 82.7 |

Process Conditions: 280 PSIG, 1000 GHSV, 195°C, $H_2/CO$ ~2/1

Catalyst: 12% $C_o$ - 1% $Re/T_iO_2$

(*1.93MPa)

## Claims

1. A hydrocarbon synthesis process comprising reacting hydrogen and carbon monoxide, in the presence of catalyst containing cobalt on a titania support or a titania-containing support, in a slurry bubble column with the Peclet number being at least 3 but not greater than 10.

2. The process of claim 1, wherein the catalyst further contains a promoter metal, preferably in a weight ratio relative to cobalt of at least 0.05/1.

3. The process of claim 2, wherein the promoter is rhenium.

4. The process of claim 3, wherein the weight ratio of rhenium is 0.1:1 to 1:1.

5. The process of any one of claims 1 to 4, wherein the catalyst is slurried in a wax having a boiling point above 700°F (371.1°C).

6. The process of claim 5, wherein the wax boils in the range of from 700-1025°F (371.1 to 551.7°C).

10

7. The process of claim 5 or claim 6, wherein the wax is produced by a hydrocarbon synthesis process employing a cobalt or cobalt/rhenium on a titania or titania containing support.

8. The process of any one of claims 1 to 7, wherein cobalt is present in an amount of at least 5 wt%.

9. The process of any one of claims 1 to 8, wherein the reaction conditions include temperatures of from 160-360°C, pressures of 80-600 psig (0.5516 to 4.137 MPa), and hydrogen to carbon monoxide ratios of from 1.5/1 to 2.5/1.

10. The process of any one of claims 1 to 9, wherein the reaction conditions include temperatures of 190-230°C, and pressures of from 150-350 psig (1.034 to 2.413 MPa).

**Patentansprüche**

1. Kohlenwasserstoffsyntheseverfahren, bei dem Wasserstoff und Kohlenmonoxid in Gegenwart eines Katalysators, der Kobalt auf einem Titandioxidträger oder einem Titandioxid-enthaltenden Träger enthält, in einer Suspensionsblasensäule umgesetzt werden, wobei die Peclet-Zahl mindestens 3, aber nicht mehr als 10 beträgt.

2. Verfahren nach Anspruch 1, bei dem der Katalysator außerdem ein Promotermetall enthält, vorzugsweise in einem Gewichtsverhältnis relativ zu Kobalt von mindestens 0,05/1.

3. Verfahren nach Anspruch 2, bei dem der Promoter Rhenium ist.

4. Verfahren nach Anspruch 3, bei dem das Gewichtsverhältnis von Rhenium relativ zu Kobalt 0,1:1 bis 1:1 ist.

5. Verfahren nach einem der Ansprüche 1 bis 4, bei dem der Katalysator in einem Wachs mit einem Siedepunkt über 371°C (700°F) suspendiert ist.

6. Verfahren nach Anspruch 5, bei dem das Wachs im Bereich von 371,1 bis 551,7°C (700 bis 1025°F) siedet.

7. Verfahren nach Anspruch 5 oder Anspruch 6, bei dem das Wachs nach einem Kohlenwasserstoffsyntheseverfahren hergestellt ist, das Kobalt oder Kobalt/Rhenium auf einem Titandioxidträger oder Titandioxid-enthaltenden Träger verwendet.

8. Verfahren nach einem der Ansprüche 1 bis 7, bei dem Kobalt in einer Menge von mindestens 5 Gew.% vorhanden ist.

9. Verfahren nach einem der Ansprüche 1 bis 8, bei dem die Reaktionsbedingungen Temperaturen von 160 bis 360°C, Drücke von 0,5516 bis 4,137 MPa (80 bis 600 psig) und Wasserstoff-zu-Kohlenmonoxid-Verhältnisse von 1,5/1 bis 2,5/1 einschließen.

10. Verfahren nach einem der Ansprüche 1 bis 9, bei dem die Reaktionsbedingungen Temperaturen von 190 bis 230°C und Drücke von 1,034 bis 2,413 MPa (150 bis 350 psig) einschließen.

**Revendications**

1. Procédé de synthèse d'hydrocarbures consistant à faire réagir de l'hydrogène et du monoxyde de carbone en présence d'un catalyseur contenant du cobalt sur un support de dioxyde de titane ou un support contenant du dioxyde de titane, dans un colonne à bulles en suspension, l'indice de Peclet étant d'au moins 3 sans être supérieur à 10.

2. Procédé selon la revendication 1, dans lequel le catalyseur contient par ailleurs un métal promoteur, de préférence selon un rapport pondéral par rapport au cobalt d'au moins 0,05/1.

3. Procédé selon la revendication 2, dans lequel le promoteur est le rhénium.

4. Procédé selon la revendication 3, dans lequel le rapport pondéral du rhénium est de 0,1:1 à 1:1.

5. Procédé selon l'une quelconque des revendications 1 à 4, dans lequel le catalyseur est mis en suspension dans une paraffine ayant un point d'ébullition supérieur à 700 ° F (371,1 ° C).

6. Procédé selon la revendication 5, dans lequel la paraffine bout dans la plage de 700 à 1025 ° F (371,1 à 551,7 ° C).

7. Procédé selon la revendication 5 ou 6, dans lequel la paraffine est produite par un procédé de synthèse d'hydrocarbures mettant en oeuvre du cobalt ou un alliage cobalt/rhénium sur un support de dioxyde de titane ou un support contenant du dioxyde de titane.

8. Procédé selon l'une quelconque des revendications 1 à 7, dans lequel le cobalt est présent à raison d'au moins 5 % en poids.

9. Procédé selon l'une quelconque des revendications 1 à 8, dans lequel les conditions réactionnelles comprennent des températures de 160 à 360 ° C, des pressions de 0,5516 à 4,137 MPa (80 à 600 psig) et des rapports de l'hydrogène au monoxyde de carbone de 1,5/1 à 2,5/1.

10. Procédé selon l'une quelconque des revendications 1 à 9, dans lequel les conditions réactionnelles comprennent des températures de 190 à 230 ° C et des pressions de 1,034 à 2,413 MPa (150 à 350 psig).

FIG. I

FIG. 2